# FASCICULE DE BREVET EUROPEEN

(11) **EP 1 285 669 B1**
(45) Date de publication et mention de la délivrance du brevet: **25.10.2006**
(21) Numéro de dépôt: 01402461.6
(22) Date de dépôt: 26.09.2001
(51) Int. Cl.: A61L 9/01, A61L 9/04, F24F 3/16

(54) **Procédé de dépollution ou d'odorification d'air**
Verfahren zur Reinigung oder Deodorisation von Luft
Method for depollution or deodorisation of air

(30) Priorité: 21.08.2001 FR 0111019
(43) Date de publication de la demande: 26.02.2003
(73) Titulaire: SOCIETE COOL S.a.r.l., F-07800 La Voulte sur Rhône (FR)
(72) Inventeur: Berrebi, Georges, 26270 Cliousclat (FR)
(74) Mandataire: Benoist, François

(56) Documents cités:
- EP-A- 0 411 206
- EP-A- 0 483 848
- JP-A- 5 317 398
- US-A- 4 735 358
- US-A- 5 154 893
- US-A- 5 782 409

## Description

La demanderesse développe des produits dans le domaine de l'environnement, du traitement de l'air.

Dans les aéroports, les hôpitaux, les grandes bibliothèques, les immeubles (type gratte-ciel ou autres) ou pour des applications industrielles, souvent l'air est chimiquement filtré.

Il convenait donc dans l'art antérieur de donner une bonne odeur à l'air. Le brevet américain US-A-5154893 décrit des agencements qui permettent de faire circuler l'air à odorer à travers une enceinte qui renferme un produit qui libère une bonne odeur, ce produit n'étant toutefois pas défmi. Le brevet américain US-A-5782409 décrit un type d'enceinte qui , placé dans un endroit pollué, libère une bonne odeur. Le produit qui libère une bonne odeur est essentiellement un odorant botanique qui préalablement à sa diffusion a été introduit par absorption sur un solide de type chlorure de sodium et matériaux cellulosiques.

EP0483848 décrit un procédé de dépollution de l'air et/ou d'odorification d'air consistant à faire circuler l'air au travers d'une cassette renfermant un agent de dépollution et/ou un agent d'odorification de l'air. L'agent d'odorification comporte un agent micro ou macroporeux tel que du papier filtre, imprégné par un parfum.

US4735358 présente un procédé et l'appareil permettant de le mettre en oeuvre pour la libération contrôlée d'un agent de dépollution de l'air et/ou d'un agent d'odorification. Les substances volatiles peuvent être aussi des substances aromatiques ou antimicrobiennes.

JP5317398 décrit la préparation d'une boule d'alumine activée ou d'alumine-silice imprégnée d'un parfum pour l'odorification de l'air.

La présente invention est un perfectionnement de l'art antérieur et décrit une méthode améliorée d'odorification de l'air ambiant à l'aide d'agents particuliers. L'invention concerne également la composition de ces agents.

Selon la présente invention, à l'aide de catalyseurs et/ ou d'adsorbants appropriés, on supprime dans cet air qui circule, les odeurs et les agents polluants (provenant notamment de processus de fabrication, des hydrocarbures imbrûlés, des fumées de cigarette et/ ou d'arômes de produits divers).

Sur le plan chimique, il s'agit de supprimer les molécules délétères (organiques ou non) contenant du soufre, de l'azote, et/ ou des composés oxygénés.

Ces catalyseurs et/ ou adsorbants peuvent être contenus dans des cassettes ou des installations type vrac, lesquelles sont placées de manière adéquate dans la circulation d'air.
La demanderesse fabrique et/ ou commercialise ce type de produit sous les noms de XMA99, XMA 250, XMA 500, XMA 255FS, DOHYL (marques déposées) etc... Tous ces produits peuvent être mélangés à du charbon actif.

L'objet de la présente invention consiste à placer dans l'air qui circule, après l'avoir purifié (ou désodorisé) des dispositifs (type cassette ou similaire) contenant des agents ou produits qui vont dépolluer l'air et/ ou lui donner ou lui redonner une odeur.
L'invention concerne aussi l'ensemble constitué par les dits dispositifs et les nouveaux produits que la demanderesse développe pour son "odorification".

**Pour "l'odorification"**, il s'agit d'obtenir un produit à base d'un support micro ou macro poreux par exemple du type charbon actif, alumine, silice, silice alumine, zéolithe sur lequel on imprègne tout ou partie de son volume poreux par au moins une huile essentielle ou au moins une solution d'huile essentielle, une diffusion d'odeurs. L'alumine activée est le support préféré.
Les produits ainsi obtenus ont un volume poreux d'au moins 45, de préférence au moins 50 cc/ 100 grammes ( 0,5 cm3/g) voire plus et une surface spécifique allant de 200 à 1200 m²/ gramme ; on peut occuper le volume poreux de sorte que les huiles essentielles contenues et imprégnées dans le support se rediffusent lentement.

Il est connu que chaque huile essentielle a un rôle sur la psychologie des comportements. Ce serait un moyen d'entretenir une atmosphère qui correspondrait mieux à l'état d'esprit des personnes qui respirent cet air.
On peut associer aux huiles essentielles lors de leur imprégnation au moins un produit bactéricide et/ ou fongicide.

Afin de doser et/ ou équilibrer les quantités d'huile essentielle émises dans l'air à odorifier on agit sur la rhéologie du système support/ huile par des procédés et/ou traitements spéciaux par exemple du type ajout de produits enrobants ou gélifiants qui vont limiter et/ou contrôler l'émission des molécules odorantes de l'huile.

Si l'air qui circule à travers ces produits (support poreux imprégné complètement ou pas) est humide, le temps pendant lequel l'odeur est extraite sera plus long. Sinon, si l'air est sec, on peut imaginer un dispositif permettant de réimprégner le support in ou ex situ.

Pour ce qui concerne plus particulièrement le support, ce support est de préférence une alumine activée. A titre d'exemple non limitatif si on l'imprègne de l'alumine activée (billes ou extrudés par exemple) par une huile essentielle dont on connaît parfaitement le spectre moléculaire qui a été analysé par chromatographie en phase gazeuse couplée d'un spectrophotomètre de masse. Cette alumine activée imprégnée par une huile essentielle est immergée dans un récipient contenant de l'eau. Comme l'alumine a plus d'affinité pour l'eau qu'elle n'en a pour l'huile essentielle, cette dernière se trouve éjectée (relarguée) et va flotter à la surface de l'eau. Cette huile essentielle, surnageant, est analysée par chromatographie en phase gazeuse, couplée avec un spectre de masse ; dans le cas spécifique de l'alumine activée, les deux spectres de la phase initiale et de la phase désorbée sont sensiblement identiques. On peut donc dire que l'alumine activée se caractérise par sa grande neutralité vis à vis des huiles essentielles, ce qui n'est pas le cas de corps poreux comme par exemple l'attapulgite (à base de magnésium) ou de la sépiolite (silicate de magnésie) qui sont utilisées par exemple comme bases pour litières de chat ou produits équivalents.

La présente invention concerne donc également une alumine activée imprégnée d'au moins une huile essentielle en vue de l'utilisation de cette alumine et de cette huile comme agent d'odorification.

## Revendications

1. Procédé d'odorification d'air consistant à faire circuler l'air à travers au moins un dispositif de type cassette ou dispositif similaire ledit dispositif renfermant un agent d'odorification, lequel agent contient une alumine activée possédant un volume poreux d'au moins 0,5 cm3/g et une surface spécifique comprise entre 200 et 1200 m²/ gramme, ladite alumine étant imprégnée au moins en partie par au moins une huile essentielle ou une solution d'huile essentielle.

2. Procédé selon la revendication 1 dans lequel on ajoute à l'huile essentielle ou à la solution d'huile essentielle, au moins un produit bactéricide ou fongicide.

3. Procédé selon l'une des revendications 1 et 2 dans lequel on ajoute dans l'alumine activée, qui renferme l'huile essentielle ou la solution d'huile essentielle, un produit enrobant ou gélifiant.

4. Alumine activée utilisable pour l'odorification d'air selon l'une des revendications 1 à 3, possédant un volume poreux d'au moins 0,5 cm3/g et une surface spécifique comprise entre 200 et 1200 m2/g et comportant en outre un produit enrobant ou gélifiant, laquelle alumine est imprégnée d'au moins une huile essentielle ou d'une solution d'huile essentielle, ladite huile essentielle contenant au moins un produit bactéricide ou fongicide.

## Claims

1. A method for scenting air, consisting of causing air to move through at least one cassette or the like type device, said device comprising a scenting agent, said agent containing an activated alumina having a pore volume of at least 0.5 cm³/g and a specific surface area in the range 200 to 1200 m²/gram, said alumina being at least partially impregnated with at least one essential oil or essential oil solution.

2. A method according to claim 1, in which at least one bactericidal or fungicidal substance is added to the essential oil or essential oil solution.

3. A method according to claim 1 or claim 2, in which a coating or gelling substance is added to the activated alumina which comprises the essential oil or essential oil solution.

4. An activated alumina for use in scenting air according to one of claims 1 to 3, having a pore volume of at least 0.5 cm³/g and a specific surface area in the range 200 to 1200 m²/g and further comprising a coating or gelling substance, said alumina being impregnated with at least one essential oil or essential oil solution, said essential oil containing at least one bactericidal or fungicidal substance.

## Patentansprüche

1. Verfahren zum Wohlriechendmachen von Luft, bestehend aus dem Zirkulierenlassen der Luft durch mindestens eine Vorrichtung vom Typ Kassette oder ähnliche Vorrichtung, wobei die besagte Vorrichtung einen Wirkstoff zum Wohlriechendmachen umschließt, welcher Wirkstoff eine aktivierte Tonerde enthält, welche ein Porenvolumen von mindestens 0,5 Kubikzentimeter/Gramm und eine spezifische Oberfläche zwischen 200 und 1200 m²/Gramm besitzt, wobei die besagte Tonerde zumindest teilweise mit mindestens einem ätherischen Öl oder einer Lösung des ätherischen Öls imprägniert ist.

2. Verfahren nach dem Anspruch 1, bei welchem man dem ätherischen Öl oder der Lösung mit ätherischem Öl mindestens einen bakteriziden oder fungiziden Stoff hinzufügt.

3. Verfahren nach einem der Ansprüche 1 und 2, bei welchem man der aktivierten Tonerde, welche das ätherische Öl oder die Lösung mit ätherischem Öl umschließt, einen einkapselnden oder gelierenden Stoff hinzufügt.

4. Aktivierte Tonerde, verwendbar zum Wohlriechendmachen von Luft gemäß einem der Ansprüche 1 bis 3, besitzend ein Porenvolumen von mindestens 0,5 Kubikzentimeter/Gramm und eine spezifische Oberfläche zwischen 200 und 1200 m²/Gramm und zusätzlich umfassend einen einkapselnden oder gelierenden Stoff, welche Tonerde mit mindestens einem ätherischen Öl oder einer Lösung mit ätherischen Ölen imprägniert ist, wobei das besagte ätherische Öl mindestens einen bakteriziden oder fungiziden Stoff enthält.
